Europäisches Patentamt

European Patent Office

Office européen des brevets

(11) Publication number: **0 276 836**
**A2**

## (12) EUROPEAN PATENT APPLICATION

(21) Application number: 88101184.5

(22) Date of filing: 27.01.88

(51) Int. Cl.⁴: **A61K 6/08** , **A61L 27/00**

(30) Priority: 27.01.87 JP 16431/87

(43) Date of publication of application:
**03.08.88 Bulletin 88/31**

(84) Designated Contracting States:
**DE FR GB**

(71) Applicant: **ASAHI KOGAKU KOGYO KABUSHIKI KAISHA**
**36-9, Maeno-cho 2-Chome Itabashi-ku Tokyo 174(JP)**

Applicant: **Mizutani, Yu**
**No. 26-24-A4, Shimizu 3-chome Suginami-ku Tokyo(JP)**

(72) Inventor: **Mizutani, Yu**
**No. 26-24-A4 Shimizu 3-chome Suginami-ku Tokyo(JP)**
Inventor: **Ichitsuka, Takeshi c/o Asahi Kogaku Kogyo K.K.**
**No. 36-9, Maeno-cho 2-chome Itabashi-ku Tokyo(JP)**

(74) Representative: **Hansen, Bernd, Dr.rer.nat. et al**
**Hoffmann, Eitle & Partner Patentanwälte Arabellastrasse 4 D-8000 München 81(DE)**

(54) Implant material and method of producing the same.

(57) An implant material for bone or soft tissue reconstruction and regeneration is composed of a sintered calcium phosphate evenly dispersed within a silicone elastomer. After hardening, the mixture can be easily shaped or formed as needed to match or conform to existing tissues. Actual bone regrowth is promoted by insuring surface availability of sintered calcium phosphate and sufficient porosity. Bone ingrowth can be assisted through the provision of conduction pores. The calcium phosphate preferably constitutes 30 - 75% by weight of the final product.

EP 0 276 836 A2

# IMPLANT MATERIAL AND METHOD OF PRODUCING THE SAME

## BACKGROUND OF THE INVENTION

The present invention relates to an implant material for use, for example, in the field of dental surgery, orthopedics, and the like, for filling a bone defect portion caused by an operation for osteoma, alveolar pyorrhea, or the like, or caused by a fracture, and relates to a method of producing such an implant material.

Conventionally, for filling bone defect portions caused by diseases developed in the jaw, in the oral cavity, or in the facial area, or caused as a result of the treatment of the above-mentioned diseases, materials including various kinds of metals and high molecular weight compounds have been used to recover the function and shape of the original bone tissue. However, with the above-mentioned materials, compatibility with the living tissue is low, and osteoconduction and osteoinductivity cannot be expected. Accordingly those materials have been used only for temporary reconstruction.

Recently, attention has been given to materials of a calcium phosphate group, represented by hydroxyapatite, as an implant material, which material exhibits osteoconductivity. Clinical applications of such implant materials have been developed in the region of the jaw and oral cavity for bone supplementation including the development of an artificial tooth root, artificial jaw bone, and so on. The hydroxyapatite material, however, has disadvantages in that the hydroxyapatite is weak in mechanical strength, so that it is inevitably used together with a metal plate or the like in clinical applications, and in that the hydroxyapatite is poor in workability, making it difficult to shape the hydroxyapatite during an operation. Recently, therefore, studies have suggested combining hydroxyapatite with a metal or high molecular weight compound so as to increase the mechanical strength of the hydroxyapatite and facilitates shaping without deteriorating osteoconductivity. For example, a composite material in which the outside of a metal core is covered with melted powder of hydroxyapatite by thermal spraying has been proposed (Japanese Kokai No. 52-82893). However, composite materials have not yet been provided which are satisfactory for use as an implant material.

## SUMMARY OF THE INVENTION

An object of the present invention is therefore to provide an implant material and a method of producing an implant material which is high in tissue affinity, so that the material can be expected to promote natural bone regrowth when the material is used as a bone-substitute, and which is easy to shape or cut.

According to a first aspect of the present invention, the implant material is characterized in that powder of sintered calcium phosphate is scattered in a continuous phase of silicone elastomer.

According to a second aspect of the present invention, the implant material is characterized in that powder of sintered calcium phosphate is scattered in a continuous phase of silicone elastomer and conduction pores are formed so as to extend from a surface of the silicone elastomer to the inside thereof.

According to a third aspect of the present invention, the method of producing an implant material is characterized by the steps of mixing a powder of sintered calcium phosphate with unhardened silicone elastomer; stirring the powder and the silicone elastomer into a mixture; molding the mixture into a predetermined shape; and thermally hardening the silicone elastomer.

According to a fourth aspect of the present invention, the method of producing an implant material is characterized by the steps of mixing a powder of sintered calcium phosphate and a water-soluble filler with unhardened silicone elastomer; stirring the silicone elastomer, the powder, and the water-soluble filler to form a mixture; molding the mixture into a predetermined shape; thermally hardening the silicone elastomer; and melting or dissolving the water-soluble filler by means of an extraction liquid to thereby form conduction pores.

As described above, the implant material according to the present invention is made of a complex composed of sintered calcium phosphate and silicone elastomer such that a fine powder of sintered calcium phosphate is surrounded by a continuous phase of silicone. Accordingly the implant material is advantageous in that it can be molded into any given shape owing to the good workability of the silicone elastomer. The silicone elastomer has substantial fluidity in the unhardened state thereof, and the implant material can be easily mechanically cut even after being hardened. Further, with respect to the compatibility with a living body, not only the calcium phosphate but silicone elastomer originally have high compatibility with a living body, so that the above-mentioned complex can form a newborn which can be united with tissues similarly to a simple substance of calcium phosphate. Further, since conduction pores are formed so as to extend from the surface of the material to the inside thereof, living cells may come into the conduction pores so as to form new bone tissue. By adjusting the porosity of the implant, it is possible to adjust the configuration of the implant material within a wide range from a spongy state to an osteoid state corresponding to the condition of the living tissues into which the implant material is embedded.

## BRIEF DESCRIPTION OF THE DRAWINGS

The present invention will be described hereunder more in detail, with reference to the attached drawings, in which:

Fig. 1 is a diagram showing the results of cell adhesion tests performed on the implant material obtained through an example according to the present invention;

Fig. 2 shows a formation of newborn recognized in the surroundings of complexes obtained according to Example 2; and

Fig. 3 shows a formation of newborn recognized in the surface of complexes obtained according to Example 2.

## DETAILED DESCRIPTION OF THE PREFERRED EMBODIMENTS

The first implant material according to the present invention is characterized in that a powder of sintered calcium phosphate is scattered in a continuous phase of silicone elastomer. The implant material has suitable elasticity, and when the content of the sintered calcium phosphate is selected to be not less than 70% by weight, the implant material exhibits impermeability to X-rays similarly to a natural bone.

According to the present invention, as the calcium phosphate, any known compound of a calcium phosphate group can be utilized. Preferable examples of the calcium phosphate group compound include hydroxyapatite $(Ca_{10}(PO_4)_6(OH)_2)$, fluorapatite $(Ca_{10}(PO_4)_6F_2)$, chlorapatite $(Ca_{10}(PO_4)_6Cl_2)$, tricalcium phosphate $(Ca_3(PO_4)_2)$, and tetracalcium phosphate $(Ca_4O(PO_4)_2)$, and so on. The sintered powder of the above-mentioned compounds can be obtained through the steps of sintering raw-material powder at a suitable high temperature, for example, about 1200 °C, and then crushing the sintered powder so as to adjust the grain size of the powder.

The grain size of the sintered calcium phosphate is selected preferably to be within a range of from 1 to 300 μm, more preferably within a range of from 1 to 100 μm. If the grain size is less than 1 Wm, the sealing force in the silicone elastomer is lowered, so that the calcium phosphate powder becomes apt to come off, especially at the surface of the complex. Such free calcium phosphate powder in a living body may accelerate the appearance of macrophage to thereby cause a biologically undesirable environment. If the grain size is selected to be larger than 300 μm, the maximum content of the calcium phosphate in the complex is lowered, so that in the implant material, the physical properties become unstable and the biological characteristics such as osteoconductivity and cell adhesion are deteriorated. Further, the grain size of the calcium phosphate need not be uniform, but may rather be made to vary so that particles of different sizes are mixed to increase the content of calcium phosphate in the complex to thereby improve the biological affinity of the implant material.

The content of the sintered calcium phosphate powder is selected preferably to be within a range of from 30 to 75% by weight. If the content of the powder is selected to be less than 30% by weight, sufficient affinity to living tissue cannot be obtained, while if it is more than 75% by weight, the physical properties of the complex become extremely deteriorated to thereby make clinical use difficult. As a result of examinations conducted using a colony forming method to measure the cell adhesion property, which is one of the aspects of affinity with living tissues, it has been proven that when the content of the sintered calcium phosphate powder is selected to be 30% by weight or more, the affinity to living tissue can be improved, while when it reaches 70% by weight, it is only possible to obtain affinities which are substantially the same as that of a simple calcium phosphate. In animal experiments, however, when the content of the sintered calcium phosphate powder was selected to be less than 70% by weight, osteoconductivity could not be expected and the rigidity and X-ray impermeability of a natural bone could not be obtained. In consideration of the above-mentioned tests, it is considered that as a soft tissue supplement material the use of a complex containing 30 - 70% by weight of calcium phosphate is preferable, while as a bone-substitute material the use of a complex containing 70 - 75% by weight of calcium phosphate is preferable.

Further, it is preferable to use a sintered calcium phosphate powder of substantial porosity, as this effectively prevents a capsule reaction (one of the foreign-matter reaction mechanisms of living tissues) from occurring.

It is considered that when the powder of sintered calcium phosphate is mixed with the silicone ealstomer, most of the powder is embedded inside the elastomer. Accordingly, in the resultant moldings, it is impossible to sufficiently utilize the affinity of calcium phosphate for living tissues. Therefore, in a preferable embodiment according to the present invention, the surface of the molded product is processed so as to expose a part of the sintered calcium phosphate powder on the surface to thereby increase the affinity of the product.

The above-mentioned first implant material according to the present invention is produced through the steps of mixing sintered calcium phosphate powder into silicone elastomer in a liquid or putty state, preferably under sterilized conditions; sufficiently stirring the powder and the elastomer to form a mixture; molding the mixture into a predetermined shape; and heating the molded mixture at a suitable temperature; for example, in the range of 80 °C to 120 °C, so as to harden the silicone elastomer to obtain an elastic body. Further, in a more preferable embodiment, the surface of the hardened implant material is subjected to mechanical cutting or the like to thereby expose the calcium phosphate powder on the surface of the implant material.

The unhardened complex of silicone elastomer and sintered calcium phosphate can be molded freely. That is, it is possible to mold the complex into granules, strings, or the like, or into the shape of particular bone structures such as the jaw bone, by filling the complex into a mold. Further, in the case where the shape of the jaw bone is to be given to the complex, a plaster mold may be prepared in advance in accordance with artificial tooth production methods known in general dentistry, and the unhardened complex then filled into the mold and thermally hardened.

The second implant material according to the present invention is characterized in that a powder of calcium phosphate is scattered in a continuous phase of silicone elastomer and conduction pores are formed so as to extend from a surface of the silicone elastomer to the inside thereof. The implant material has sufficient elasticity and is impermeable to X-rays similarly to natural bone when the content of the sintered calcium phosphate powder is selected to be 70% by weight or more. It is possible to give the implant material a variety of shapes within a range of spongy to osteoid, corresponding to the porosity of the material. With such conduction pores, the implant material is superior in affinity when embedded in the tissues of a living body, because the passage of humor through the implant material is good, and even if a phagocyte such as macrophage adheres to the implant material, a foreign matter reaction is unlikely to be caused by macrophage and a capsule reaction is more effectively prevented from occurring, whereby superior affinity to living tissues can be obtained.

Also in the second implant material, for the same reason as that described in the above case, the grain size of calcium phosphate is selected preferably to be within a range of 1 μm to 300 μm, more preferably within a range of 1 μm to 100 μm. The content of the sintered calcium phosphate powder is preferably selected to be within a range of 30 to 75% by weight. Further, it is more preferable that the surface of the molded product be subjected to mechanical cutting so as to expose part of the sintered calcium phosphate powder on the surface. Furthermore, it is much more preferable to use a porous powder of sintered calcium phosphate.

The porosity of the implant material can be varied in accordance with the desired shape, ranging from spongy to osteoid. Generally, it is desirable to select the porosity to be within a range of from about 10 to 90%, and to select the average diameter of the pores to be within a range of from about 1 m to 500 ¯μm.

The second implant material according to the present invention is produced through the steps of mixing a sintered calcium phosphate powder and a water-soluble filler into silicone elastomer in a liquid or putty state, preferably under sterilized conditions; sufficiently stirring the powder, the water-soluble filler and the elastomer to form a mixture; molding the mixture into a predetermined shape; heating the molded mixture at a suitable temperature, for example, in a range of 80 °C to 120 °C, so as to harden the silicone elastomer to obtain an elastic body; and dipping the elastic body in an extraction liquid so as to resolve and remove the water soluble filler. More preferably, the surface of the hardened implant material is subjected to mechanical cutting or the like to thereby expose the calcium phosphate powder on the surface of the implant material. Further, the unhardened complex of silicone elastomer and sintered calcium phosphate can be molded freely, similarly to the case of the first implant material. That is, it is possible to mold the complex into granules, strings, and the like, or into special bone shapes by filling the complex in a mold.

As the water soluble filler used in the second implant material, any water soluble material can be utilized so long as it is a water-soluble substance which is inert to living tissues. For example, glycine, mannitol, glucose, and the like, may be used as the water soluble filler. In this case, as the extraction liquid, it is preferable to use water, more preferably distilled water. It is necessary to sufficiently dissolve the water soluble filler so that no filler remains. If the implant material is embedded into tissues with filler remaining in the implant material, it is considered that the remaining chemicals may change the pH around the complex to thereby change the tissue environment. In this regard, dissolved glycine is discharged from living tissues without changing the tissue environment around the implant complex even if glycine remains in the living body. Thus, it is considered that glycine is a more preferable material.

Being elastic, the first and second implant materials do not have the physical properties of natural bone. However, it is possible to reconstruct a defective bone portion through the use of the implant material together with a metal plate, or the like, when the implant material is used as an artificial bone. In this case, it is easy to perform deletion or formation processes such as trimming during an operation. Further, the implant material may be preferably used for the reconstruction of soft tissues, because the implant material may be formed into various shapes in advance or during an operation.

The present invention will be described more specifically with respect to the following specific examples thereof.

Example 1

Fine particles (having grain size of 1-100 μm) of porous hydroxyapatite calcium phosphate sintered at 1200 °C were mixed, under sterilized conditions, into silicone elastomer containing polydimethyl siloxane as a main component to obtain four mixtures in which the content of the sintered calcium phosphate was selected to be 10, 30, 50 and 70% by weight, respectively. Each of the mixtures was stirred sufficiently, and heated at 100 °C for 4 hours to thereby be hardened. The surfaces of some of the complexes were cut mechanically so as to expose the particles of the hydroxyapatite.

The thus obtained complexes were examined for tissue adhesive properties using a colony formation method. HeLa S3 cells and L cells were used to examine the surface tissue adhesion, and cell-suspension liquids of those cells were prepared using MEM medium containing 10% calf serum. The cell-suspension liquids were poured on complexes applied to respective bottom surfaces of petri dishes. The cells were incubated at 37°C in a 5% $CO_2$ incubator, and the state of the cells were observed three days later. 300 Hela S3 cells were incubated on the complex, and the colony forming rate one week later was examined.

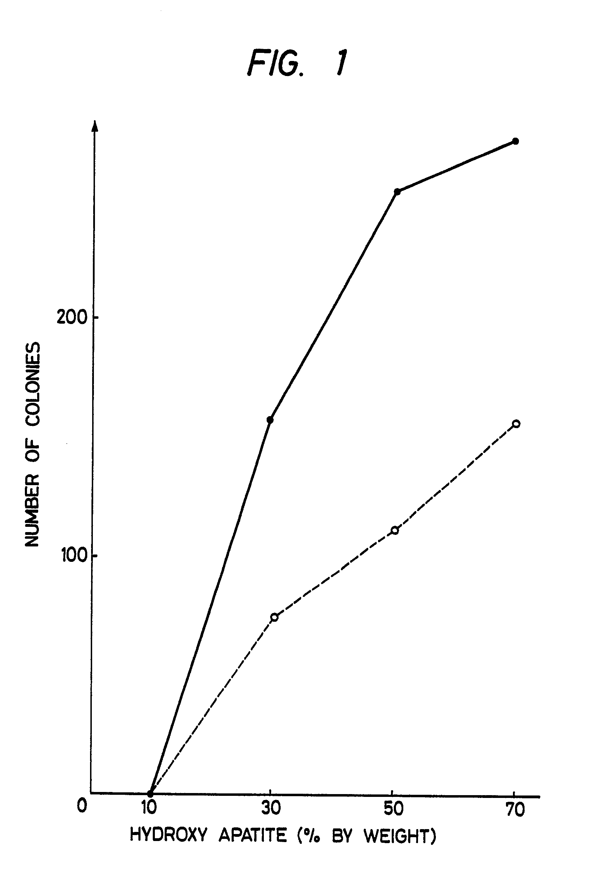

As a result, as shown in Fig. 1, it was found that the number of adhering cells increases as the content of apatite in the complex increases, and that if the content of apatite exceeds 30% by weight, the number of colonies increases rapidly.

Especially, in the group in which the complexes were subjected to surface processing, almost all of the cells produced colonies when the apatite content reached 70% by weight, and the cell adhesive property was remarkably improved. In the drawing, the abscissa represents the content of apatite, the ordinate represents the number of colonies, line A shows the results with respect to the surface-treated group, and line B shows the result with respect to the non-surface-treated group.

Further, a selected complex containing hydroxyapatite at 70% by weight, the surface of which was subjected to mechanical cutting was applied onto the leg bone of a rabbit. The rabbit was sacrificed one month later and a non-decalcified section was produced after fixing by a common method. The non-decalcified section was dyed with toluidine blue and observed. Newborn was seen between the leg bone and the complex, and the newborn was in direct contact with the surface of the complex, proving the osteoconductivity of the complex.

Example 2

Fine particles (having a grain size of 10 - 100 μm) of porous calcium phosphate sintered at 1200 °C were mixed together with glycine (having a grain size of 50 -200 μm), under sterilized conditions, into silicone elastomer containing polydimethyl siloxane as a main component to obtain four mixtures in which the content of the sintered calcium phosphate was selected to be 10, 30, 50, and 70% by weight respectively. The thus obtained mixtures were stirred sufficiently, heated for four hours at 100 °C so as to be hardened, and then dipped and left in distilled water in which an antibiotic agent was dissolved so that the glycine contained in the complex material could be eluded out. The additive rate of glycine was adjusted so that the resultant complexes had a porosity of 50% - 60%.

The cell adhesion properties of the thus obtained complexes were tested by a colony formation method similarly to the case of Example 1. As a result, good cell adhesion properties were recognized in Example 2, similarly to the case of Example 1.

Of the thus obtained complexes having conduction pores, one containing hydroxyapatite at 70% by weight, the surface of which was subjected to mechanical cutting was applied onto the leg bone of a rabbit. similarly to the case of Example 1, newborn was observed and osteoconductivity was recognized.

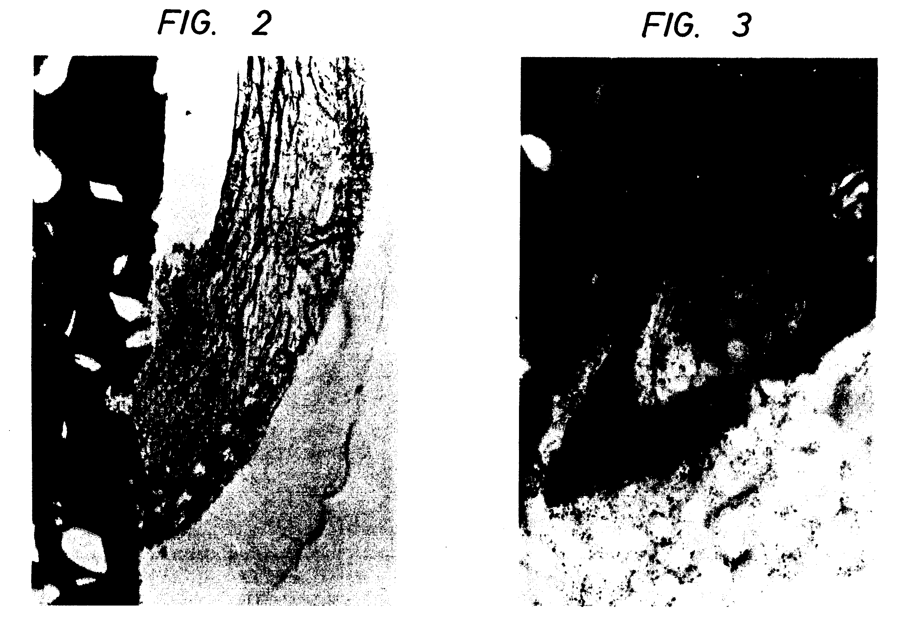

Specifically, a hole having a diameter of 5 mm was formed by drilling in a leg bone or femur of a rabbit and the porous complex was embedded in the hole. The rabbit was killed one month later, and non-deliming thin cut pieces were produced from the region of the hole. The non-deliming thin cut pieces were H-E dyed and bone formation in the surroundings of the complex was observed and examined. As a result, a newborn was recognized in the surroundings of the complex, and, particularly at a part adjacent to bone cortex, hyperplasia or proliferation of newborn was so remarkable that the newborn was in contact directly with the periphery of the complex as shown in Fig. 2. Further, at a part of the hole opened in the surface of the complex, the newborn entered the inside of the hole to fill the hole, and, furthermore, formation of newborn was observed even in the pores of the complex which communicated with the surface hole as shown in Fig. 3. However, no newborn was recognized in closed pores of the complex which did not communicate with the surface hole.

As described above, the implant material according to the present invention is advantageous, in that its affinity to living tissues is high and bone addition can be expected in and around the implant material when the implant material is used as a bone-substitute. Shape forming or cutting can be made easy in accordance with known dental techniques so that the implant material may be made applicable to numerous uses including corrective surgery for bone deformation or defects, especially in jaw or facial bone reconstruction.

## Claims

1. An implant material for use as a bone or soft tissue substitute, comprising a powder of sintered calcium phosphate dispersed within a continuous phase of silicone elastomer.

2. An implant material according to claim 1, in which a part of said powder of sintered calcium phosphate is exposed on a surface of said implant material.

3. An implant material according to claim 1, in which an average grain size of said powder of sintered calcium phosphate is within a range of 1 $\mu$m to 300 $\mu$m.

4. An implant material according to claim 1, in which said powder of sintered calcium phosphate is present in said material in a range of 30 - 75% by weight.

5. An implant material for use as a bone or soft tissue substitute, comprising: a powder of calcium phosphate dispersed in a continuous phase of silicone elastomer to form a complex, and conduction pores formed in said complex so as to extend from a surface of said complex to the interior thereof.

6. An implant material according to claim 5, in which a part of said powder of sintered calcium phosphate is exposed on the surface of said implant material.

7. An implant material according to claim 6, in which an average grain size of said powder of sintered calcium phosphate is within a range of 1 $\mu$m to 300 $\mu$m.

8. An implant material according to claim 5, in which said powder of sintered calcium phosphate is present in said complex in a range of 30 - 75% by weight.

9. An implant material according to claim 5, in which the porosity of said conduction pores is within a range of 10% to 90%.

10. An implant material according to claim 5, in which an average size of said conduction pores is within a range of from 1 $\mu$m to 500 $\mu$m.

11. A method of producing a bone or soft tissue implant material, comprising the steps of: mixing a powder of sintered calcium phosphate into an unhardened silicone elastomer; stirring said powder and said silicone elastomer to form a mixture; molding said mixture into a predetermined shape; and thermally hardening said silicone elastomer.

12. A method as claimed in claim 11, further comprising mechanically processing a surface of said silicone elastomer after thermal hardening to thereby expose a part of said powder of sintered calcium phosphate on the surface of said silicone elastomer.

13. A method of producing a bone or soft tissue implant material, comprising the steps of: mixing a powder of sintered calcium phosphate and a water-soluble filler into an unhardened silicone elastomer; stirring said silicone elastomer, said powder, and said water-soluble filler to form a mixture; molding said mixture into a predetermined shape; thermally hardening said silicone elastomer; and dissolving and eluting out said water-soluble filler by means of an extraction liquid, to form conduction pores.

14. A method as claimed in claim 13, further comprising the step of mechanically processing a surface of said silicone elastomer after thermal hardening to expose a part of said powder of sintered calcium phosphate on the surface of said silicone elastomer.

15. A method as claimed in claim 13, in which glycine is used as said water-soluble filler, and water is used as said extraction liquid.

# FIG. 1

NUMBER OF COLONIES

200

100

0    10        30        50        70

HYDROXY APATITE (% BY WEIGHT)

FIG. 2

FIG. 3

0 276 836